# EUROPÄISCHE PATENTSCHRIFT

(11) **EP 1 655 045 B1**
(45) Veröffentlichungstag und Bekanntmachung des Hinweises auf die Patenterteilung: **26.03.2008**
(21) Anmeldenummer: 05022481.5
(22) Anmeldetag: 14.10.2005
(51) Int. Cl.: A61M 3/02

(54) **Vorrichtung zur Belüftung eines Darmspülgerätes**
Venting device for an intestinal irrigation apparatus
Dispositif pour ventilation d'un appareil d'irrigation intestinale

(30) Priorität: 30.10.2004 DE 202004016919 U
(43) Veröffentlichungstag der Anmeldung: 10.05.2006
(73) Patentinhaber: Eich, Helmut, 72805 Lichtenstein (DE)
(72) Erfinder: Eich, Helmut, 72805 Lichtenstein (DE)
(74) Vertreter: Ludewig, Rita

(56) Entgegenhaltungen:
- WO-A-87/01596
- DE-A1- 4 429 068
- DE-U1- 29 610 226

## Beschreibung

Die Erfindung betrifft eine Vorrichtung zur Belüftung eines Darmspülgerätes insbesondere zur Anwendung im medizinischen Bereich.

Bekannte Darmspülgeräte bilden bei der Darmspülung ein Vakuum, damit sie eine Saugfunktion ausüben können.

Die Einrichtungen sind im allgemeinen mit einem Wasserzuflußstutzen und einem Wasserabflußstutzen ausgerüstet, wobei das zufließende Wasser über einen Zuflußschlauch und ein Spekulum in den Darm geleitet und das mit Darmbestandteilen angereicherte Wasser aus dem Darm über das Spekulum und einen Abflußschlauch in das Darmspülgerät abgesaugt werden kann. Die Bedienperson kann die abgesaugten Darmbestandteile über ein an dem Darmspülgerät von außen sichtbares Sichtrohr begutachten.

Beim Spülvorgang kommt es während einer Darmspülung sehr häufig vor, daß die mit den Darmbestandteilen belastete, abgesaugte Flüssigkeit im Sichtrohr, stehen bleibt und der Absaugvorgang unterbrochen wird.

Um den Spülvorgang fortsetzen zu können, muß bei jeder Unterbrechung außerhalb des Gerätes der Abflußschlauch vom Stutzen leicht angehoben oder gar entfernt werden, um das Sichtrohr zu belüften. Dabei ist es nicht zu vermeiden, daß neben der Unterbrechung des Spülvorganges mit Darmbestandteilen angereichertes Wasser austritt. Die Liege, der Patient und die Bedienperson werden beschmutzt, es sind unangenehme, vor allem unhygienische Reinigungsvorgänge und Arbeitsabläufe zu Fortsetzung der Spülung erforderlich und es tritt eine erhebliche Geruchsbelästigung auf.

Für den Patienten hat das weitere unangenehme Auswirkungen, in dem er sich verkrampft und sich die ohnehin unangenehme Prozedur insgesamt zeitlich verlängert, was wiederum den dafür erforderlichen Zeit- und Kostenaufwand erhöht.

Aus der G 94 03 578.4 ist ein Belüftungsventil für ein Unterdruck-Abwassersystem bekannt, wobei von einem Ventilgehäuse ausgegangen wird, das mindestens eine Öffnung für Zuluft aufweist sowie mit einem anhebbaren Ventilkolben ausgestattet ist, der von einem Ventilsitz mittels eines Unterdruckschlauchs einen Unterdruck zuführen kann. Dabei besteht das Belüftungsventil aus einem zweiteiligen Ventilgehäuse, das durch eine Membran unterteilt ist und in dem der Ventilkolben einen Raumteil für die Zuluft und einen Raumteil für einen Unterdruckanschluß durchdringt. Das Belüftungsventil ist durch einen komplizierten konstruktiven Aufbau gekennzeichnet und somit kostenaufwendig.

Aus der DE 296 10 226 U1 ist auch ein Darmspülgerät mit einer Erweiterung bekannt, die aus einem Entlüftungsventil besteht, das als Zwischenrohrstück zwischen der Schlauchkupplung und dem Abflußschlauch außerhalb des Gerätes angeordnet ist und dazu dient im Darm gebildete Gase aus dem Spülkreislauf zu entfernen um ihn zu entlüften. Die Lösung sieht in einer Variante ein einfaches Ventil mit einem Stopfen vor, der leicht geöffnet und wieder verschlossen werden kann. Beim öffnen des Ventilstopfens kann die Darmluft entweichen. Diese Lösung hat den Nachteil, daß das Ventil außerhalb des Gehäuses angeordnet ist, wodurch beim öffnen nicht nur übelrichende Darmgase in den Behandlungsraum entweichen, sondern auch mit Darmbestandteilen belastetes Wasser austreten kann was wiederum zu Verunreinigungen führt.

Die zweite Variante dieser Lösung sieht vor, daß ebenfalls außerhalb des Gerätes direkt am Abflußstutzen ein Entlüftungsventil angeschlossen ist, das ein Schaltventil und einen Entlüftungsstutzen mit Abgasrohr aufweist und über einen Bedienungshebel zu öffnen und zu schließen ist. Am unteren Teil des Abgasrohrstücks ist ein Überlaufstutzen angeordnet. Beim öffnen des Entlüftungsventils entweichen übelrichende Gase in den Behandlungsraum und dabei mit austretende, mit Darmbestandteilen belastete Wasserbestandteile über den Überlaufstutzen in einen dazu aufgestellten Behälter. Diese Lösung ist kompliziert im Aufbau, erfordert zusätzliche Anbauarbeiten an dem Darmspülgerät und hat wiederum unangenehme Begleichterscheinungen (insbesondere Geruchsbelästigung) zur Folge. Außerdem ist die nachträgliche Ausstattung des Darmspülgerätes eine sehr kostenaufwendige Angelegenheit.

Aus der WO 87/01596 A ist ein Darmspülgerät bekannt, die dem Grundaufbau der bekannten Darmspülgeräte gleicher Kategorie entspricht. Die offenbarte Erfindung beinhaltet eine Vervollkommnung des Verfahrens und des Gerätes zur Darmspülung, dahingehend, daß eine neues Spekulum mit verschiedenen Formen und Zu- und Abgängen entwickelt wurde, dessen Funktion die Patentansprüche 1 bis 11 und dessen Aufbau die Ansprüche 12 bis 22 beanspruchen. In den Ansprüchen 23 bis 41 werden weiter Bestandteile des Gerätes beansprucht, die unter anderem die Regelung der Warm- und Kaltwasserzufuhr und deren Mischung, die automatische Druckregelung der Wasserzufuhr in den Darm des Patienten, die Beimengung von Injektionsflüssigkeiten während der Behandlung, und die Anordnung eines Sichtrohrs mit Lupe vorsehen. Es ist auch die Entnahme von Proben aus dem Abflußschlauch vorgesehen, die mit Hilfe eines von außen bedienbaren Dreiwegeventils in einen an der Außenwand des Gehäuses angeordneten, mit einem, durch eine Abdeckvorrichtung mit einem offenen Entlüftungsrohr verschlossenen, Behälter zu leiten sind, bevor die verunreinigten Abwasserproben aus dem Gerät in das Abwasserrohr geleitet werden. Damit die Proben aus dem Abflußschlauch in den Behälter einfließen können, muß die in diesem befindliche Luft verdrängt werden, weshalb an der Verschlußvorrichtung ein Entlüftungsrohr angeordnet ist, über das die Luft in die Umwelt entweichen kann. Eine Belüftung des geschlossenen Kreislaufs, die in der gesamten Beschreibung weder vorgesehen noch erwähnt ist, ist über diesen Weg nicht realisierbar, weil die in dem Behälter befindliche Luft durch den Einlauf von Flüssigkeit aus dem Abflußschlauch in den Behälter über das Entlüftungsrohr nach außen verdrängt wird. Das Eindringen von Luft in den Kreislauf ist auf diese Weise unmöglich.

Es war deshalb Aufgabe eine Vorrichtung zur Belüftung eines Darmspülgerätes zu entwickeln, die durch eine einfache Handhabung der Bedienperson auszulösen ist, einen einfachen konstruktiven Aufbau aufweist, im Gehäuse Platz findet und kostengünstig ist und die bisherigen unangenehmen Begleiterscheinungen wie Geruchsbelästigungen, zeitaufwendige Reinigungsarbeiten sowie erheblichen zusätzlichen Zeitaufwand für den Darmspülvorgang insgesamt vermeidet.

Die Aufgabe wird durch eine Vorrichtung zur Belüftung eines Darmspülgerätes mit den kennzeichnenden Merkmalen des Anspruchs 1 gelöst.

Vorteilhaft ist der einfache konstruktive Aufbau der Vorrichtung, der eine kostengünstige Herstellung und Montage innerhalb des Darmspülgerätes gestattet.

Besonders hervorzuheben ist die Anordnung der Vorrichtung zur Belüftung innerhalb des Gehäuses des Darmspülgerätes zwischen der Schlauchkupplung des Abflußschlauchs und dem Sichtrohr und die Anordnung des Bedienknopfes der Vorrichtung an der Gehäuseaußenwand des Darmspülgerätes. Diese Anordnung hat zur Folge, daß beim Stillstand des Spülvorganges, der durch die Bedienperson von außen im Sichtrohr erkennbar ist, die Bedienperson nur kurz den Bedienknopf drücken muß, wodurch dem Saugsystem Luft zugeführt wird, ohne daß übelrichende Gase oder mit Darmbestandteilen belastetes Wasser austreten kann. Der Spülvorgang kann ohne zusätzliche Arbeitsvorgänge fortgesetzt werden. Von besonderem Vorteil ist die einfache Handhabung für die Bedienperson, die Zeiteinsparung bei der Darmspülung und die Vermeidung der für den Patienten und die Bedienperson bisher aufgetretenen unangenehmen Begleiterscheinungen wie Geruchsbelästigung und Reinigungsarbeiten.

Die Anordnung der Muffe mit Axialbohrung nach Anspruch 2 zwischen der Schlauchkupplung des Abflußschlauches und dem Sichtrohr ermöglicht darüber hinaus über einen kurzen Weg eine Luftzuführung im Sichtrohr und damit die sofortige Auflösung des Staus. Ein Austreten von Gasen und von mit Darmbestandteilen belastetem Wasser ist nicht möglich, wodurch auch keine Geruchsbelästigung und Verunreinigungen auftreten können.

Von erheblichem Vorteil ist die einfache konstruktive Ausführung des Belüftungsventils nach den Ansprüchen 3 bis 6. Das Belüftungsventil besteht aus einem Zylinder, einem Gewindebolzen, einer Druckfeder mit Dichtung und einem Bedienknopf und kann mittels seinem Außengewinde auf einfache weise mit der erfindungsgemäßen Vorrichtung zur Belüftung im Gehäuse verbunden werden. Das Ventil hat lediglich die Aufgabe bei Auftreten eines Staus im Sichtrohr durch kurzen Knopfdruck die Belüftung des Sichtrohrs zu gewährleisten und so den Stau sofort zu beheben und ohne weitere Handgriffe die sofortige Fortführung des Spülvorganges zu gewährleisten.

Die besonders hervorzuhebende einfache und kurzzeitige Handhabung zur Auslösung und Beendigung des Belüftungsvorganges nach den Ansprüchen 7 und 8 durch Knopfdruck von außen spricht für sich.

Die Erfindung soll nachstehend anhand eines Ausführungsbeispiels näher beschrieben werden. Die Zeichnungen zeigen dabei in
- Fig. 1: die Vorderansicht der montierten Vorrichtung zur Belüftung mit Schlauchkupplung und Sichtrohr,
- Fig.2: einen Ausschnitt der Vorderansicht der montierten Vorrichtung zur Belüftung mit Schlauchkupplung und Sichtrohr eingebaut in ein Gehäuse eines Darmspülgerätes,
- Fig.3: vereinfachte Schnittdarstellung des Aufbaus des Belüftungsventils eingebaut in die Verschraubung des Luftschlauchs
- Fig.4: vereinfachte Schnittdarstellung der Muffe verbunden über eine Verschraubung mit dem Luftschlauch und verbunden mit dem eingestecktem Sichtrohr.

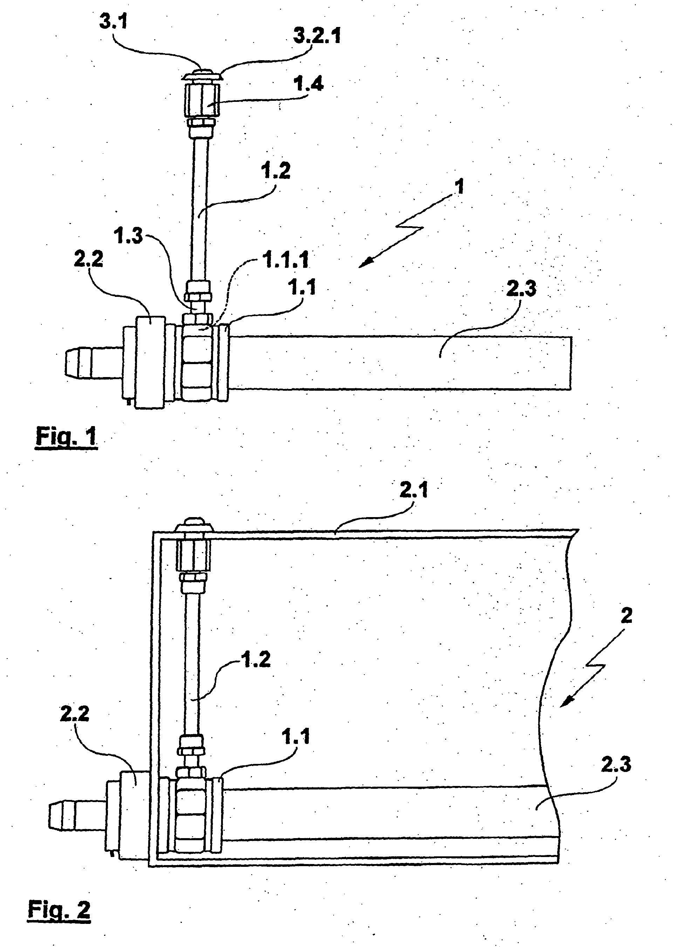

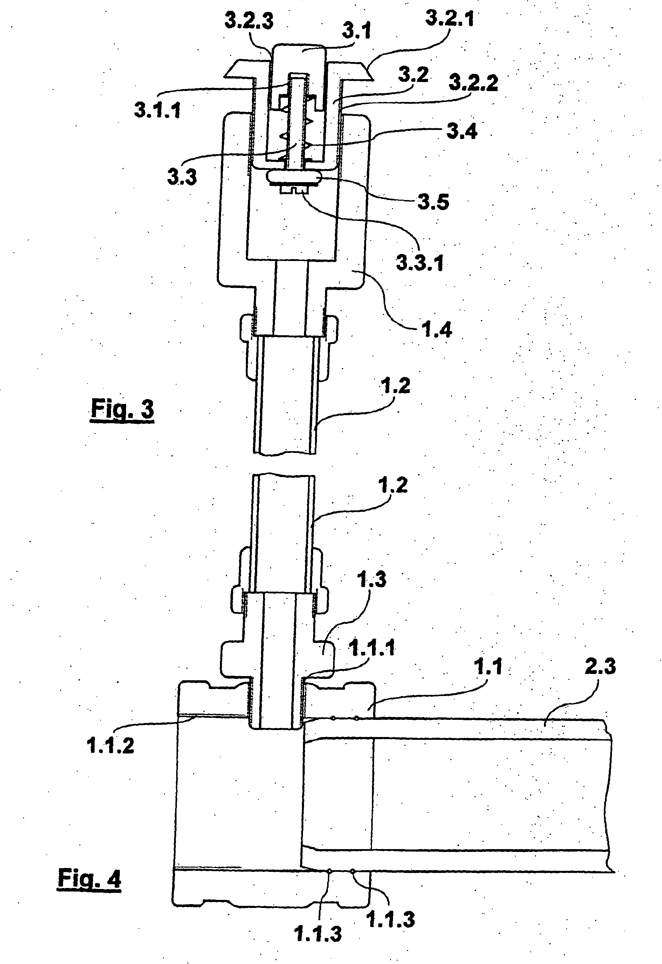

Im Ausführungsbeispiel, gemäß Fig.1, wurde eine Vorrichtung 1 zur Belüftung eines Darmspülgerätes 2 konstruiert, die vorzugsweise aus einer Muffe 1.1 mit einer Horizontalbohrung 1.1.1 mit einseitigem Innengewinde 1.1.2 und gegenüberliegender Steckverbindung mit Dichtungsringen 1.1.3 besteht. Die Muffe 1.1 gemäß Fig.4 wird mit ihrer einen Seite mittels Innengewinde 1.1.2 auf die von außen durch die Gehäusewand des Gehäuses 2.1 geführte Schlauchkupplung 2.2 eines Abflußschlauchs von innen aufgeschraubt. In die gegenüberliegende Seite der Muffe 1.1 wird das Sichtrohr 2.3 mit einem leicht konischen Ende eingesteckt, wobei die Dichtheit dieser Verbindung beispielsweise durch O- Ringe in der Muffe 1.1 gewährleistet ist. In der Horizontalbohrung 1.1.1 der Muffe 1.1 wird eine Verschraubung 1.3 angeordnet und mit einem Luftschlauch 1.2 verbunden. Das zweite gegenüberliegende Ende des Luftschlauchs 1.2 wird über eine zweite Verschraubung 1.4 mit dem Belüftungsventil 3 über dessen Außengewinde 3.2.2 verbunden.

Das Belüftungsventil 3 nach Fig.3 besteht vorzugsweise aus einem Zylinder 3.2 mit einem Bund 3.2.1 einem Außengewinde 3.2.2 und einer Durchgangsbohrung 3.2.3, die an einer aus dem Gehäuse 2.1 herausragenden Seite eine größere Öffnung und an der in das Gehäuse 2.1 gerichteten Seite eine kleinere Öffnung aufweist. Zur Montage des Belüftungsventils 3 wird vorzugsweise auf einen Gewindebolzen 3.3 mit Kopf 3.3.1 eine Dichtung 3.5 aufgesetzt. Der Gewindebolzen 3.3 wird mit der aufgesetzten Dichtung 3.5 in die kleine Öffnung der Durchgangsbohrung 3.2.3 gesteckt. Von der gegenüberliegenden Öffnung der Durchgangsbohrung 3.2.3 im Zylinder 3.2 wird eine Druckfeder 3.4 auf den Gewindebolzen 3.3 geschoben und mit einem Bedienknopf 3.1 mit Innengewinde 3.1.1 verbunden.

Das vormontierte Belüftungsventil 3 wird, gemäß Fig.2, von außen durch die Gehäuswand des Gehäuses 2.1 gesteckt, wobei der Bund 3.2.1 einen stabilen Sitz des Belüftungsventils 3 auf der Gehäusewand gewährleistet. Von innen wird das Belüftungsventil 3 über sein Außengewinde 3.2.2. mit der Verschraubung 1.4 des Luftschlauches 1.2 verbunden, wodurch der Belüftungskreislauf geschlossen wird.

### Aufstellung der verwendeten Bezugszeichen

- 1: Vorrichtung zur Belüftung,
- 1.1: Muffe,
- 1.1.1: Horizontalbohrung,
- 1.1.2: Innengewinde,
- 1.1.3: O- Ringe
- 1.2: Luftschlauch,
- 1.3: Verschraubung,
- 1.4: Verschraubung
- 2.: Darmspülgerät,
- 2.1: Gehäuse,
- 2.2: Schlauchkupplung,
- 2.3: Sichtrohr,
- 2.4: Abflußschlauch,
- 3.: Belüftungsventil,
- 3.1: Bedienknopf
- 3.1.1: Innengewinde,
- 3.2: Zylinder,
- 3.2.1: Bund,
- 3.2.2: Außengewinde,
- 3.2.3: Durchgangsbohrung,
- 3.3: Gewindebolzen,
- 3.3.1: Kopf des Gewindebolzens,
- 3.4: Druckfeder,
- 3.5: Dichtung,

## Patentansprüche

1. Vorrichtung (1) zur Belüftung eines Darmspülgerätes (2), unter anderem aufweisend ein Gehäuse (2.1) in dem am Gehäuseeingang eine Schlauchkupplung (2.2) mit einem sich anschließenden Sichtrohr (2.3) und eine sich daran anschließende Schlauchkupplung am Gehäuseausgang angeordnet ist, durch die innerhalb eines geschlossenen Kreislaufes kontaminiertes Wasser aus dem Darm eines Patienten in einen Abfluß außerhalb des Gehäuses (2.1) geleitet wird, **dadurch gekennzeichnet,**
**daß** die Vorrichtung (1) zur Belüftung des Darmspülgerätes (2) aus einer Muffe (1.1) mit einer Horizontalbohrung (1.1.1), einem Innengewinde (1.1.2), O- Ringen (1.1.3) einem Luftschlauch (1.2) mit beidseitig angeordneten Verschraubungen (1.3 und 1.4), und einem Belüftungsventil (3) mit dem Bedienknopf (3.1) gebildet ist und in dem Gehäuse (2.1) zwischen der Schlauchkupplung (2.2) und dem Sichtrohr (2.3) angeordnet werden kann und durch den am Gehäuse (2.1) anbringbaren Bedienknopf (3.1) von außen zu betätigen ist.

2. Vorrichtung (1) zur Belüftung eines Darmspülgerätes (2), nach Anspruch **1, dadurch gekennzeichnet, daß** im Gebrauchszustand die Muffe (1.1) die Schlauchkupplung (2.2) eines Abflußschlauchs mit dem Sichtrohr (2.3) des Darmspülgerätes (2) verbindet und der Luftschlauch (1.2) über die Verschraubung (1.3) einerseits mit der Horizontalbohrung (1.1.1) der Muffe (1.1) und mit der Verschraubung (1.4) andererseits mit dem Belüftungsventil (3) und dem Bedienknopf (3.1) der Vorrichtung (1) verbunden ist.

3. Vorrichtung (1) zur Belüftung eines Darmspülgerätes (2), nach Anspruch 1, **dadurch gekennzeichnet, daß** das Belüftungsventil (3) ein Druckventil ist.

4. Vorrichtung (1) zur Belüftung eines Darmspülgerätes (2), nach Anspruch 1, **dadurch gekennzeichnet, daß** das Belüftungsventil (3) aus einem Zylinder (3.2) mit einseitigem Bund (3.2.1) und Außengewinde (3.2.2) und einer längs gerichteten Durchgangsbohrung (3.2.3), einem Gewindebolzen (3.3) mit Kopf (3.3.1), einer Druckfeder (3.4), einer Dichtung (3.5) sowie aus dem Bedienknopf (3.1) mit Innengewinde (3.1.1) gebildet ist und über das Außengewinde (3.2.2) mit der Verschraubung (1.4) des Luftschlauchs (1.2) mit der Vorrichtung (1) verbunden ist.

5. Vorrichtung (1) zur Belüftung eines Darmspülgerätes (2), nach Anspruch 4, **dadurch gekennzeichnet, daß** die längs gerichtete Durchgangsbohrung (3.2.3) des Zylinders (3.2) zwei unterschiedlich große Öffnungen aufweist, wobei die Öffnung an der Bundseite größer ist als die Öffnung an der dem Bund gegenüberliegenden Seite.

6. Vorrichtung (1) zur Belüftung eines Darmspülgerätes (2), nach Anspruch 4, **dadurch gekennzeichnet, daß** der Gewindebolzen (3.3) mit der aufgesteckten Dichtung (3.5) in die kleinere Öffnung des Zylinders (3.2) und die Druckfeder (3.4) sowie der Bedienknopf (3.1) in die gegenüberliegende größere Öffnung des Zylinders (3.2) gesteckt sind und das freie Ende des Gewindebolzens (3.3) mit dem Innengewinde (3.1.1) des Bedienknopfes (3.1) verschraubt ist, wobei im Gebrauchszustand das Belüftungsventil (3) von außen durch die Wand des Gehäuses (2.1) der Darmspüleinrichtung (2) gesteckt und von innen mit der Verschraubung (1.4) des Luftschlauches (1.2) der Vorrichtung (1) verbunden ist.

7. Vorrichtung (1) zur Belüftung eines Darmspülgerätes (2), nach Anspruch 4, **dadurch gekennzeichnet, daß** durch Betätigung des Bedienknopfes (3.1) im Gebrauchszustand die Druckfeder (3.5) im Belüftungsventil (3) zusammengedrückt, Luft von außen über das Belüftungsventil (3) und den Luftschlauch (1.2) in die Muffe (1.1) und von dort direkt in das Sichtrohr (2.2) eindringt und das Sichtrohr (2.2) belüftet ist.

8. Vorrichtung (1) zur Belüftung eines Darmspülgerätes (2), nach Anspruch 4, **dadurch gekennzeichnet, daß** sich durch Loslassen des Bedienknopfes (3.1) im Gebrauchszustand die Druckfeder (3.5) entspannt und das Belüftungsventil (3) schließt, wobei die Luftzufuhr in die Vorrichtung (1) und somit im Sichtrohr (2.2) der Darmspüleinrichtung (2) wieder unterbrochen ist.

## Claims

1. Device (1) for the ventilation of a gastrointestinal rinsing unit (2), featuring among other things a housing (2.1), wherein at the housing entry a hose coupling (2.2) with a connected sighting tube (2.3), and a hose coupling which is connected at the housing exit are provided and through which contaminated water is channelled from the intestines of a patient through a closed circuit to a drain outside the housing (2.1), **characterised in that**
the device (1) for the ventilation of the gastrointestinal rinsing unit (2) is composed of a sleeve (1.1) with a horizontal boring (1.1.1), an inner thread (1.1.2), O-rings (1.1.3), an air hose (1.2) with screw connections (1.3 and 1.4) arranged on both sides, and a ventilation valve (3) with the operating button (3.1), and which can be arranged in the housing (2.1) between the hose coupling (2.2) and the sighting tube (2.3) and is to be operated from the exterior via the operating button (3.1) that may be attached to the housing (2.1).

2. Device (1) for the ventilation of a gastrointestinal rinsing unit (2), according to Claim 1, **characterised in that**, in the state of use, the sleeve (1.1) connects the hose coupling (2.2) of a drain hose with the sighting tube (2.3) of the gastrointestinal rinsing unit (2) and the air hose (1.2) is connected with the horizontal boring (1.1.1) of the sleeve (1.1) via the screw connection (1.3) on one side and is connected with the ventilation valve (3) and the operating button (3.1) of the device (1) via the screw connection (1.4) on the other side.

3. Device (1) for the ventilation of a gastrointestinal rinsing unit (2), according to Claim 1, **characterised in that** the ventilation valve (3) is a pressure valve.

4. Device (1) for the ventilation of a gastrointestinal rinsing unit (2), according to Claim 1, **characterised in that** the ventilation valve (3) is composed of a cylinder (3.2) with a one-sided collar (3.2.1) and outer thread (3.2.2) and a lengthwise-oriented through boring . (3.2.3), a thread bolt (3.3) with head (3.3.1), a pressure spring (3.4), a seal (3.5), as well as an operating button (3.1) with inner thread (3.1.1) and that it is connected to the device (1) via the outer thread (3.2.2) with the screw connection (1.4) of the air hose (1.2).

5. Device (1) for the ventilation of a gastrointestinal rinsing unit (2), according to Claim 4, **characterised in that** the lengthwise-oriented through boring (3.2.3) of the cylinder (3.2) features two openings of differing size, whereby the opening on the collar side is larger than the opening located on the side opposite the collar.

6. Device (1) for the ventilation of a gastrointestinal rinsing unit (2), according to Claim 4, **characterised in that** the thread bolt (3.3) with the applied seal (3.5) is inserted in the smaller opening of the cylinder (3.2), and the pressure spring (3.4) as well as the operating button (3.1) is inserted in the opposite, larger opening of the cylinder (3.2), and that the free end of the thread bolt (3.3) is screwed together with the inner thread (3.1.1) of the operating button (3.1), whereby in the state of use the ventilation valve (3) is inserted from the exterior through the wall of the housing (2.1) of the gastrointestinal rinsing unit (2) and is connected from the interior with the screw connection (1.4) of the air hose (1.2) of the device (1).

7. Device (1) for the ventilation of a gastrointestinal rinsing unit (2), according to Claim 4, **characterised in that**, through activation of the operating button (3.1) in the state of use, the pressure spring (3.5) in the ventilation valve (3) compresses and air is vented from the exterior to the sleeve (1.1) via the ventilation valve (3) and the air hose (1.2) and then penetrates from the sleeve (1.1) directly into the sighting tube (2.2), thereby ventilating the sighting tube (2.2).

8. Device (1) for the ventilation of a gastrointestinal rinsing unit (2), according to Claim 4, **characterised in that**, through release of the operating button (3.1) in the state of use, the pressure spring (3.5) relaxes and the ventilation valve (3) closes, thereby re-interrupting the air supply to the device (1) and therefore also to the sighting tube (2.2) of the gastrointestinal rinsing unit (2).

## Revendications

1. Dispositif (1) de ventilation d'un appareil d'irrigation intestinale (2), comprenant entre autres un boîtier (2.1) dans lequel, à son entrée, est disposé un raccord de tuyau flexible (2.2) suivi d'un tuyau de surveillance (2.3) et, à la suite de celui-ci, à la sortie dudit boîtier, est disposé un raccord de tuyau flexible à travers lequel de l'eau contaminée provenant de l'intestin d'un patient est acheminée à l'intérieur d'un circuit fermé dans un débouché à l'extérieur du boîtier (2.1), **caractérisé par le fait que** ledit dispositif (1) de ventilation de l'appareil d'irrigation intestinale (2) est constitué par un manchon (1.1) ayant un perçage horizontal (1.1.1), un filet intérieur (1.1.2), des joints toriques (1.1.3), par un tuyau flexible à air (1.2) comprenant des vissages (1.3 et 1.4) disposés de part et d'autre et par une soupape de ventilation (3) avec le bouton de manoeuvre (3.1), et peut être disposé dans ledit boîtier (2.1) entre le raccord de tuyau flexible (2.2) et le tuyau de surveillance (2.3) et est à actionner de l'extérieur par l'intermédiaire du bouton de manoeuvre (3.1) qui peut être monté sur ledit boîtier (2.1).

2. Dispositif (1) de ventilation d'un appareil d'irrigation intestinale (2), selon la revendication 1, **caractérisé par le fait que**, en état d'utilisation, ledit manchon (1.1) relie ledit raccord de tuyau flexible (2.2) d'un tuyau flexible de décharge audit tuyau de surveillance (2.3) de l'appareil d'irrigation intestinale (2) et que le tuyau flexible à air (1.2) est relié, d'un côté, par le vissage (1.3) au perçage horizontal (1.1.1) du manchon (1.1) et, de l'autre côté, par le vissage (1.4) à la soupape de ventilation (3) et au bouton de manoeuvre (3.1) du dispositif (1).

3. Dispositif (1) de ventilation d'un appareil d'irrigation intestinale (2), selon la revendication 1, **caractérisé par le fait que** ladite soupape de ventilation (3) est une soupape de pression.

4. Dispositif (1) de ventilation d'un appareil d'irrigation intestinale (2), selon la revendication 1, **caractérisé par le fait que** ladite soupape de ventilation (3) est constituée par un cylindre (3.2) ayant une collerette unilatérale (3.2.1) et un filet extérieur (3.2.2) et un trou de passage (3.2.3) dirigé longitudinalement, par un boulon fileté (3.3) avec une tête (3.3.1), par un ressort à pression (3.4), par un joint d'étanchéité (3.5) ainsi que par ledit bouton de manoeuvre (3.1) à filet intérieur (3.1.1), et est reliée par ledit filet extérieur (3.2.2) avec le vissage (1.4) du tuyau flexible (1.2) au dispositif (1).

5. Dispositif (1) de ventilation d'un appareil d'irrigation intestinale (2), selon la revendication 4, **caractérisé par le fait que** ledit trou de passage dirigé longitudinalement (3.2.3) du cylindre (3.2) présente deux orifices à dimension différente, l'orifice situé du côté de la collerette étant plus grand que l'orifice situé du côté opposé à la collerette.

6. Dispositif (1) de ventilation d'un appareil d'irrigation intestinale (2), selon la revendication 4, **caractérisé par le fait que** ledit boulon fileté (3.3) avec le joint d'étanchéité (3.5) rapporté est introduit dans l'orifice plus petit du cylindre (3.2) et que le ressort à pression (3.4) ainsi que le bouton de manoeuvre (3.1) sont introduits dans l'orifice opposé plus grand du cylindre (3.2) et que l'extrémité libre du boulon fileté (3.3) est vissée avec le filet intérieur (3.1.1) du bouton de manoeuvre (3.1) ; en état d'utilisation, la soupape de ventilation (3) étant introduite de l'extérieur à travers la paroi du boîtier (2.1) du dispositif d'irrigation intestinale (2) et étant reliée de l'intérieur au vissage (1.4) du tuyau flexible à air (1.2) du dispositif (1).

7. Dispositif (1) de ventilation d'un appareil d'irrigation intestinale (2), selon la revendication 4, **caractérisé par le fait que**, en actionnant ledit bouton de manoeuvre (3.1) en état d'utilisation, le ressort à pression (3.4) dans la soupape de ventilation est comprimé, de l'air passe de l'extérieur via la soupape de ventilation (3) et le tuyau flexible à air (1.2) et pénètre dans ledit manchon (1.1) et de là directement dans le tuyau de surveillance (2.2), et le tuyau de surveillance (2.2) est ventilé.

8. Dispositif (1) de ventilation d'un appareil d'irrigation intestinale (2), selon la revendication 4, **caractérisé par le fait que**, en relâchant le bouton de manoeuvre (3.1) en état d'utilisation, le ressort à pression (3.4) se détend et la soupape de ventilation (3) se ferme, l'amenée d'air dans le dispositif (1) et ainsi dans le tuyau de surveillance (2.2) du dispositif d'irrigation intestinale (2) étant à nouveau interrompue.
